Europäisches Patentamt

European Patent Office

Office européen des brevets

(11) Veröffentlichungsnummer: **0 359 075**
**A1**

## EUROPÄISCHE PATENTANMELDUNG

(21) Anmeldenummer: **89116248.9**

(22) Anmeldetag: **02.09.89**

(51) Int. Cl.5 **C12P 19/04**

(30) Priorität: **08.09.88 DE 3830469**

(43) Veröffentlichungstag der Anmeldung:
**21.03.90 Patentblatt 90/12**

(84) Benannte Vertragsstaaten:
**ES GR**

(71) Anmelder: **Chemische Fabrik Grünau GmbH**
**Robert-Hansen-Strasse 1**
**D-7918 Illertissen(DE)**

(72) Erfinder: **Schnepp, Walter**
**Hans-Holbein-Strasse 15**
**D-7918 Illertissen(DE)**
Erfinder: **Sander, Andreas**
**Karlsbader Strasse 19**
**D-7918 Illertissen(DE)**
Erfinder: **Schuster, Gregor**
**Ludwigstrasse 31**
**D-7910 Neu-Ulm(DE)**
Erfinder: **Fiala, Franz**
**Auer Strasse 55**
**D-7918 Illertissen(DE)**
Erfinder: **Fuchs, Bertram**
**Schulstrasse 12**
**D-7919 Buch(DE)**

(54) **Verfahren zur Herstellung pulverförmiger, wasserauswaschbarer, enzymatisch abgebauter Heteropolysaccharide.**

(57) Es wird ein Verfahren zur Herstellung pulverförmiger, wasserauswaschbarer, enzymatisch abgebauter Heteropolysaccharide beschrieben.

EP 0 359 075 A1

## Verfahren zur Herstellung pulverförmiger, wasserauswaschbarer, enzymatisch abgebauter Heteropolysaccharide

Die Erfindung betrifft ein Verfahren zur Herstellung pulverförmiger, wasserauswaschbarer, enzymatisch abgebauter Heteropolysaccharide sowie die Verwendung derselben als Schlichtemittel.

Gewebe bestehen aus Schuß- und Kettfäden. Während die Schußfäden beim Webvorgang nur einmal kurzzeitig stark beansprucht werden, erfahren die Kettfäden eine Langzeitbelastung, die in der Hauptsache durch die Scheuerwirkung der Führungsorgane der Webmaschinen hervorgerufen wird. Dieser enormen Beanspruchung sind jedoch normalerweise die Kettgarne nicht gewachsen. Sie werden daher vor dem Webvorgang mit Lösungen von Schlichtemitteln behandelt. Diese Schlichtemittel verleihen den Garnen Festigkeit, Scheuerbeständigkeit und Geschmeidigkeit.

Schlichtemittel sind im allgemeinen in Wasser teilweise oder vollständig lösliche Substanzen, wie native oder modifizierte Stärken, Celluloseether, Eiweißprodukte, Polyvinylalkohole, Polyacrylate ode löslich gemachte Tamarindenkernmehle (siehe beispielsweise in "Ullmanns Encyclopädie der technischen Chemie", Band 23, Seiten 12 - 17, Verlang Chemie Weinheim (1983)).

Nach dem Weben hat die Schlichte ihre Aufgabe erfüllt. Da sie bei den weiteren Veredlungsstufen stört, muß sie entweder durch einfaches Auswaschen mit Wasser oder durch die Einwirkung von Enzymen oder Chemikalien und anschließendem Auswaschen mit Wasser vollständig entfernt werden.

Schlichten, die durch einfaches Auswaschen mit Wasser entfernt werden können, werden vorzugsweise eingesetzt. Das bedeutet, daß diese Schlichtemittel nach ihrer Verwendung weitgehend in das Abwasser gelangen. Aus Gründen des Umweltschutzes ist daher eine möglichst vollständige biologische Abbaubarkeit für diese Produkte zu fordern. Die Abbaubarkeit entspricht bei den üblicherweise als voll auswaschbar eingesetzten synthetischen oder halbsynthetischen Schlichtemitteln, beispielsweise Stärkederivaten, Carboxymethylcellulosen, Polyvinylalkoholen und/oder Polyacrylaten nicht dieser Forderung. Schlichtemittel auf Basis von Samenkernmehlen, insbesondere Tamarindenkernmehlen, sind in biologischen Kläranlagen jedoch vollständig abbaubar und deswegen als mit Wasser auswaschbare Schlichtemittel besonders gut geeignet.

Tamarindenkernmehl, das aus dem Endosperm der entschälten Samen des Tamarindenbaumes (Tamarindus indica) durch Mahlen gewonnen wird, ist jedoch als solches wegen seiner geringen Löslichkeit in Wasser und der hohen Viskosität wäßriger gekochter Lösungen als Schlichtemittel ungeeignet. So besitzt beispielsweise eine 5 Gew.-%ige wäßrige Lösung von Tamarindenkernmehl nach 30 Minuten Rühren bei 95 bis 98 °C eine Viskosität zwischen 2 000 und 3 200 mPas, gemessen bei 80 °C auf dem Brookfield-Viskosimeter, Modell RVT bei 20 Umdrehungen pro Minute und Spindel 1. Zur Erhöhung der Löslichkeit und zur Erniedrigung der Viskosität von Tamarindenkernmehl in Wasser muß dieses daher abgebaut, d.h. depolymerisiert, werden. Da mit Säuren oder Laugen abgebaute Tamarindenkernmehle hohe Salzgehalte besitzen, die die Verarbeitbarkeit sowie die Eigenschaften der Gewebe negativ beeinflussen, werden üblicherweise Tamarindenkernmehle enzymatisch abgebaut. Dieser Abbau wird in wäßrigen Suspensionen mit Tamarindenkernmehlgehalten zwischen 2 und 25 Gew.-% durchgeführt (siehe beispielsweise Chemical Abstract 73, 110815j (1970)). Bei der Herstellung pulverförmiger Produkte aus Suspensionen fallen jedoch hohe Trocknungskosten an.

Die Aufgabe der Erfindung bestand daher in der Entwicklung eines Verfahrens zur Herstellung pulverförmiger, wasserauswaschbarer, enzymatisch abgebauter Heteropolysaccharide, bei welchem die Trocknungskosten deutlich reduziert sind.

Überraschenderweise wurde gefunden, daß Heteropolysaccharide in Pulverform in Gegenwart von Wassergehalten bis zu etwa 40 Gew.-% enzymatisch abgebaut werden können. Im Vergleich zu suspendierten Heteropolysacchariden erfolgt der enzymatische Abbau pulverförmiger Heteropolysaccharide mit deutlich geringeren Wassermengen.

Gegenstand der Erfindung ist daher ein Verfahren zur Herstellung pulverförmiger, wasserauswaschbarer, enzymatisch abgebauter Heteropolysaccharide, welches dadurch gekennzeichnet ist, daß man Heteropolysaccharide mit 0,5 bis 3,0 Gew.-% Enzymen und 20 bis 60 Gew.-% Wasser, jeweils bezogen auf Heteropolysaccharidmenge, bei Temperaturen zwischen 15 und 30 °C vermischt, die Mischungen bei Temperaturen zwischen 35 und 65 °C intensiv rührt, anschließend die Enzyme bei Temperaturen zwischen 60 und 100 °C desaktiviert und das Reaktionsgemisch trocknet und vermahlt.

Vorzugsweise werden Heteropolysaccharide, bezogen auf Heteropolysaccharidmenge, mit 2 bis 3 Gew.-% Enzymen und 30 bis 50 Gew.-% Wasser bei Raumtemperatur, d. h. bei Temperaturen zwischen 15 und 30 °C befeuchtet und intensiv gemischt. Die gesamte Wassermenge kann den Heteropolysacchariden auf einmal oder in mehreren Teilmengen zugesetzt werden. Sofern der pH-Wert der Mischungen nicht mit dem pH-Wert der optimalen Enzymaktivität übereinstimmt, wird dieser mit Säuren, beispielsweise Phosphorsäure, oder Laugen, beispielsweise Natronlauge, auf das pH-Wert Optimum der eingesetzten Enzyme eingestellt. Anschließend werden die Mischungen auf Temperaturen zwischen 35 und 65 °C, vorzugsweise auf 55 bis 65 °C aufgeheizt und in Abhängigkeit von den Einsatzmengen der Mischungen 1 bis 10 Stunden bei dieser Temperatur vermischt.

Für das erfindungsgemäße Verfahren eignen sich als Heteropolysaccharide Tamarindenkernmehl, Guarmehl, Johannisbrotkernmehl, Mehl der Cassia occidentalis sowie Mischungen dieser Mehle, vorzugsweise Tamarindenkernmehl und/oder Guarmehl. Die Herstellung dieser Mehle ist zum Beispiel in "Handbook of water-soluble Gums and Resins", Robert L. Davidson (ed.), Kap. 6, Seite 6.2, Kap. 14, Seite 14.2 und Kap. 23, Seite 23.3, McGraw-Hill 1980 beschrieben. Das Mehl der Cassia occidentialis wird durch Mahlen des Endosperms der Cassia occidentalis Leguminosae gewonnen.

Der enzymatische Abbau der Heteropolysaccharide erfolgt vorzugsweise mit Pentosanasen, Cellulasen, Hemicellulasen, Galaktomannanasen, Proteinasen sowie Mischungen dieser Enzyme, beispielsweise mit SP 342, einem Enzymgemisch von Novo, Dänemark oder mit Multifect L 250, einem Enzymgemisch der Firma Finnsugar, Finnland.

Zur Desaktivierung der eingesetzten Enzyme werden die Reaktionsmischungen auf Temperaturen zwischen 60 und 100 °C, vorzugsweise auf Temperaturen zwischen 60 und 90 °C aufgeheizt und bei diesen Temperaturen 1 bis 7 Stunden gemischt. Um eine Verfärbung der Produkte zu verhindern, kann es in manchen Fällen vorteilhaft sein, vor Beginn der Desaktivierung den Reaktionsmischungen Wasserstoffperoxid zuzusetzen, beispielsweise 0.5 bis 5 Gew.-%, vorzugsweise 2 bis 3 Gew.-% - jeweils bezogen auf Heteropolysaccharidmenge - wäßrige 50 Gew.-%ige Wasserstoffperoxidlösung.

Anschließend wird der pH-Wert, falls erforderlich, mit Laugen, beispielsweise Natronlauge, auf 5,0 bis 8,0 eingestellt und die enzymatisch abgebauten Heteropolysaccharide getrocknet und danach auf Stift-, Hammer- oder Prallmühlen vermahlen. Die Trocknungs- und Vermahlungstemperaturen können beispielsweise zwischen 40 und 80 °C liegen.

In der vermahlenen, enzymatisch abgebauten Heteropolysacchariden liegt der Wassergehalt zwischen 1 und 20 Gew.-%, vorzugsweise zwischen 5 und 10 Gew.-%.

Die Viskosität der erfindungsgemäß hergestellten Heteropolysaccharide liegt in 5 Gew.-%igen wäßrigen Lösungen zwischen 15 und 1 000 mPas, gemessen bei einer Temperatur von 80 °C auf dem Brookfield-Viskosimeter, Modell RVT bei 20 Umdrehungen pro Minute und Spindel 1.

Weiterer Erfindungsgegenstand ist die Verwendung der nach dem erfindungsgemäßen Verfahren hergestellten pulverförmigen Heteropolysaccharide als Schlichtemittel. Die enzymatisch abgebauten Heteropolysaccharide sind salzarm und im Gegensatz zu Polyvinylalkohol auch in alkalischen Medien löslich. Durch die Entfernbarkeit der Schlichte vom Gewebe durch Auswaschen mit Wasser bei Temperaturen zwischen 60 und 90 °C erübrigt sich eine enzymatische Entschlichtung.

Die erfindungsgemäß zu verwendenden enzymatisch abgebauten Heteropolysaccharide können alleine oder in Kombination mit weiteren Schlichtemitteln, beispielsweise modifizierten Stärken, Stärkederivaten und/oder Gelatine, eingesetzt werden, wobei der Heteropolysaccharidgehalt zwischen 20 und 80 Gew.-%, vorzugsweise zwischen 50 und 70 Gew.-% liegt. Die nach dem erfindungsgemäßen Verfahren erhaltenen Heteropolysaccharide werden vorzugsweise aus wäßriger Flotte durch Foulardierung und anschließende Trocknung auf das Garn appliziert. In den wäßrigen Flotten liegt der Gehalt an enzymatisch abgebauten Heteropolysacchariden zwischen 5 und 20 Gew.-%, vorzugsweise zwischen 5 und 10 Gew.-%. In den Schlichteflotten können als weitere Bestandteile 1 bis 5 Gew.-% Schlichtefette, beispielsweise sulfatierte Fette und/oder Öle enthalten sein.

Beispiele

Die Angabe Teile bedeutet Gewichtsteile.

Beispiel 1

100 Teile Tamarindenkernmehl wurden in einem Pulvermischer vorgelegt und unter laufendem Mischwerk mit einer Lösung von 3 Teilen SP 342 (Enzymmischung von Novo, Dänemark) in 30 Teilen Wasser innerhalb von 5 bis 10 Minuten bei Raumtemperatur versetzt. Unter guter Durchmischung werden weitere 20 Teile Wasser zugegeben, auf 60 bis 65 °C aufgeheizt und bei dieser Temperatur weitere 4 Stunden gemischt. Danach wurde zur Desaktivierung des Enzymgemisches auf 85 bis 90 °C aufgeheizt und 3 Stunden bei dieser Temperatur gemischt. Anschließend wurde das Reaktionsgemisch mit Hilfe eines Wirbelschichttrockners bei 70 °C Produkttemperatur getrocknet und auf einer Stiftmühle bei 50 °C vermahlen.

Eine 5 Gew.-%ige wäßrige Lösung zeigte nach 30 minütigem Rühren bei 95 bis 100 °C eine Viskosität von 25 bis 39 mPas, bei 80 °C (gemessen an einem Brookfield-Viskosimeter Modell RVT bei 20 Umdrehungen pro Minute und Spindel 1) und einen pH-Wert von 6 bis 7.

Beispiel 2

100 Teile Tamarindenkernmehl werden in einem Pulvermischer vorgelegt und unter laufendem Mischwerk mit einer Lösung von 3 Teilen SP 342 (Enzymmischung von Novo, Dänemark) in 30 Teilen Wasser innerhalb von 5 bis 10 Minuten bei Raumtemperatur versetzt. Unter guter Durchmischung wurden weitere 10 Teile Wasser zugegeben, auf 55 bis 60 °C aufgeheizt und 4 Stunden bei dieser Temperatur gemischt. Danach wurde eine Lösung aus 2,5 Teilen wäßriger Wasserstoffperoxidlösung, 50 Gew.-%ig und 7,5 Teilen Wasser zugegeben. Zur Desaktivierung des Enzymgemisches wurde auf 80 bis 85 °C aufgeheizt und 2 Stunden bei dieser Temperatur gemischt. Anchließend wurde das Reaktionsgemisch analog Beispiel 1 getrocknet und vermahlen.

Eine 5 Gew.-%ige wäßrige Lösung zeigt nach 30 minütigem Rühren bei 95 bis 100 °C eine Viskosität von 20 bis 25 mPas, bei 80 °C (Brookfield-Viskosimeter, Modell RVT bei 20 Umdrehungen pro Minute und Spindel 1) und einen pH-Wert von 5 bis 6.

Beispiel 3

100 Teile Tamarindenkernmehl wurden in einem Pulvermischer vorgelegt und unter laufendem Mischwerk bei Raumtemperatur mit einer Lösung von 3,6 Teilen Phosphorsäure 75%ig und 30 Teilen Wasser innerhalb von 5 bis 10 Minuten versetzt. Nach einer Mischzeit von 15 Minuten wurder der pH-Wert des Mischgutes mit 4,7 bestimmt. Nach Zugabe einer Lösung aus 3 Teilen Multifect L 250 und 16 Teilen Wasser wurde das Reaktionsgemisch auf 55 bis 60 °C aufgeheizt und 6 Stunden bei dieser Temperatur gemischt. Danach wurde der pH-Wert mit einer Mischung von 3,6 Teilen Natronlauge 50%ig und 2,4 Teilen Wasser auf 7,1 eingestellt und zur Desaktivierung 1 Stunde bei 60 °C gemischt. Anschließend wurde des Reaktionsprodukt analog Beispiel 1 getrocknet und vermahlen.

Eine 5 Gew.-%ige wäßrige Lösung zeigt nach 30-minütigem Rühren bei 95 bis 100 °C eine Viskosität von 20 bis 25 mPas bei 80 °C (Brookfield-Viskosimeter Modell RVT bei 20 Umdrehungen/min und Spindel 1) und einen pH-Wert von 7,1.

**Ansprüche**

1. Verfahren zur Herstellung pulverförmiger, wasserauswaschbarer, enzymatisch abgebauter Heteropolysaccharide, dadurch gekennzeichnet, daß man Heteropolysaccharide mit 0,5 bis 3,0 Gew.-% Enzymen und 20 bis 60 Gew.-% Wasser, jeweils bezogen auf Heteropolysaccharid-Menge, bei Temperaturen zwischen 15 und 30 °C vermischt, die Mischungen bei Temperaturen zwischen 35 und 65 °C intensiv rührt, anschließend die Enzyme bei Temperaturen zwischen 60 und 100 °C desaktiviert und das Reaktionsgemisch trocknet und vermahlt.

2. Verfahren nach Anspruch 1, dadurch gekennzeichnet, daß man Heteropolysaccharide mit 2 bis 3 Gew.-% Enzymen und 30 bis 50 Gew.-% Wasser, jeweils bezogen auf Heteropolysaccharid-Menge, vermischt.

3. Verfahren nach einem beiden der Ansprüche 1 bis 2, dadurch gekennzeichnet, daß man als Heteropolysaccharide Tamarindenkernmehl, Guarmehl, Johannisbrotkernmehl, Mehl der Cassia occidentalis oder Mischungen dieser Heteropolysaccharide, vorzugsweise Tamarindenkernmehl und/oder Guarmehl einsetzt.

4. Verfahren nach einem oder mehreren der Ansprüche 1 bis 3, dadurch gekennzeichnet, daß man als Enzyme Pentosanasen, Cellulasen, Hemicellulasen, Galaktomannanasen, Proteinasen oder Mischungen dieser Enzyme einsetzt.

5. Verfahren nach einem oder mehreren der Ansprüche 1 bis 4, dadurch gekennzeichnet, daß man die Mischungen bei 55 bis 65 °C intensiv rührt.

6. Verfahren nach einem oder mehreren der Ansprüche 1 bis 5, dadurch gekennzeichnet, daß man vor der Desaktivierung den Reaktionsmischungen wäßrige Wasserstoffperoxidlösungen zusetzt.

7. Verwendung pulverförmiger, wasserauswaschbarer, enzymatisch abgebauter Heteropolysaccharide, hergestellt nach einem oder mehreren der Ansprüche 1 bis 6 als Schlichtemittel.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-3 480 511  (D.A. JONES)<br>* Anspruch 1 *<br>--- | 1 | C 12 P  19/04 |
| D,A | CHEMICAL ABSTRACTS, Band 73, Nr. 22, 30. November 1970, Seite 51, Zusammenfassungs Nr. 110815j, Columbus, Ohio, US; & IN-A-112 845 (AHMEDABAD TEXTILE INDUSTRY'S RESEARCH ASSOC.) 14-10-1970<br>* Zusammenfassung *<br>--- | 1,7 | |
| A | GB-A-1 042 438  (BLATTMANN)<br>* Seite 2, Zeilen 88-116 *<br>----- | | |

|  |
|---|
| **RECHERCHIERTE SACHGEBIETE (Int. Cl.5)** |
| C 12 P<br>C 08 B |

Der vorliegende Recherchenbericht wurde für alle Patentansprüche erstellt

| Recherchenort | Abschlußdatum der Recherche | Prüfer |
|---|---|---|
| DEN HAAG | 29-11-1989 | SOMERVILLE F.M. |